(19) Europäisches Patentamt
European Patent Office
Office européen des brevets

(11) Veröffentlichungsnummer : **0 434 708 B1**

# (12) EUROPÄISCHE PATENTSCHRIFT

(45) Veröffentlichungstag der Patentschrift :
**13.05.92 Patentblatt 92/20**

(51) Int. Cl.$^5$ : **C07K 1/02,** C07K 1/12

(21) Anmeldenummer : **89909738.0**

(22) Anmeldetag : **09.09.89**

(86) Internationale Anmeldenummer :
**PCT/EP89/01051**

(87) Internationale Veröffentlichungsnummer :
**WO 90/03387 05.04.90 Gazette 90/08**

## (54) VERFAHREN ZUR HERSTELLUNG VON DIPEPTIDEN MIT N-TERMINALEN NICHTPROTEINOGENEN AMINOSÄUREN.

(30) Priorität : **17.09.88 DE 3831717**

(43) Veröffentlichungstag der Anmeldung :
**03.07.91 Patentblatt 91/27**

(45) Bekanntmachung des Hinweises auf die Patenterteilung :
**13.05.92 Patentblatt 92/20**

(84) Benannte Vertragsstaaten :
**AT BE CH DE FR GB IT LI NL**

(56) Entgegenhaltungen :
**Liebigs Analen der Chemie, Nr. 3, March 1987, VCH Verlags gesellschaft mbH, (Weinheim, DE), B Köhler et al.: "Synthese vonDipeptidtriestern mit Phosphonoglycin als N-terminaler Komponente", pages 267-269.**

(56) Entgegenhaltungen :
**Liebigs Analen der Chemie, issue 11, November 1988, VCH Verlagsgesellschaft mbH, (Weinheim, DE), U Cchöllkopf et al.:"Asymetric synthesis of Boc-L-Val-(R)-alpha-MePro-Ome, Boc-L-Val-(R)-Pro-Ome, and of Boc-L-Val-(R)-alpha-MePhe-Ome,Ac-L-Val-(R)-alpha-MePhe-Ome and their analogues. A new strategy for the synthesis of non-proteinogenic bipeptides", pages1025-1031. See the whole article, in particular the introduction of the pages 1025-1027 and the production of compound 22, page1030**

(73) Patentinhaber : **BASF Aktiengesellschaft**
**Carl-Bosch-Strasse 38**
**W-6700 Ludwigshafen (DE)**

(72) Erfinder : **SCHÖLLKOPF, Ulrich**
**Eichenweg 5**
**W-3406 Bovenden (DE)**
Erfinder : **GROTH, Ulrich**
**Ernst-Ruhstrat-Strasse 6**
**W-3400 Göttingen (DE)**
Erfinder : **LANGE, Meinolf**
**Hünstollenstrasse 17**
**W-3401 Waake/Bösinghausen (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue Saint-Denis, 75001 PARIS

**Beschreibung**

Die vorliegende Erfindung betrifft ein Verfahren zur Herstellung von Dipeptiden, die sich von nicht-proteinogenen Aminosäuren ableiten.

Zur gezielten Synthese von Peptiden, die nicht proteinogene Aminosäuren enthalten, werden die unnatürlichen Aminosäuren unter Verwendung üblicher Schutzgruppen und Aktivierungsmethoden zum stufenweisen Aufbau der gewünschten Peptide eingesetzt.

Die eingesetzten unnatürlichen Aminosäuren werden bei dieser Strategie entweder über eine nicht enantioselektive Synthese und anschließende Racematspaltung oder mittels enantioselektiver Synthesen unter Verwendung optisch aktiver Hilfsstoffe gewonnen.

Schwierigkeiten bei der Ausbildung der Peptidbindung können besonders dann auftreten, wenn die $\alpha$-Position der unnatürlichen Aminosäure durch zwei sterisch anspruchsvolle Reste substituiert ist (Helv. Chim. Acta 69, (1986) 1153, J. Amer. Chem. Soc. 103 (1981) 6127).

Es wurde nun gefunden, daß man bestimmte Dipeptide auf einfache Weise herstellen kann.

Gegenstand der Erfindung ist ein Verfahren zur Herstellung von Dipeptiden mit N-terminalen nicht-proteinogenen Aminosäuren der Formel I

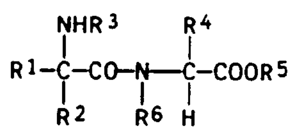 I

worin

$R^1$ eine $C_1$-$C_8$-Alkyl-, Phenyl- oder Benzylgruppe ist

$R^2$ eine $C_1$-$C_8$-Alkylgruppe, die durch -O-, -S-, -CO- oder -CO-O- unterbrochen sein kann, oder eine Phenyl- oder Benzylgruppe bedeutet,

$R^3$ ein Wasserstoffatom ist oder zusammen mit $R^2$ die Reste $-(CH_2)_3-$, $-(CH_2)_4-$ oder $-CH_2-CH=CH-CH_2-$ darstellt und

$R^4$ einen Methyl-, Isopropyl-, Isobutyl-, 2-Butyl-, t-Butyl- oder Benzylrest bedeutet,

$R^5$ eine Methyl- oder Ethylgruppe darstellt und

$R^6$ ein Wasserstoffatom oder eine $C_{1-8}$-Alkylgruppe darstellt,

wobei jedoch mindestens einer der beiden Reste $R^3$ oder $R^6$ ein Wasserstoffatom ist, welches darin besteht, daß man

a) - falls $R^6$ ein Wasserstoffatom ist - eine Verbindung der Formel II

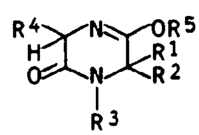 II,

worin $R^1$ bis $R^5$ die angegebene Bedeutung haben, mit Trialkyloxoniumfluoroborat zu Verbindungen der Formel III

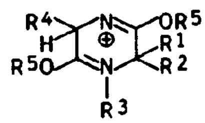 $BF_4^{\ominus}$ III,

worin $R^1$ bis $R^5$ die angegebene Bedeutung haben, umsetzt und die so erhaltenen Verbindungen mit Säure spaltet und hydrolysiert oder

b) - falls $R^3$ ein Wasserstoffatom und $R^5$ eine Ethylgruppe bedeuten - eine Verbindung der Formel IV

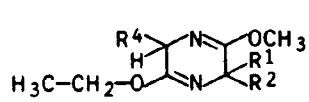 VI,

worin $R^1$, $R^2$ und $R^4$ die angegebene Bedeutung besitzen, mit einem Trialkylsilyliodid umsetzt, danach gegebenenfalls alkyliert und die so erhaltenen Verbindungen anschließend mit Säure spaltet oder c) - falls $R^3$ ein Wasserstoffatom ist - eine Verbindung der Formel VIII

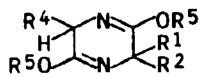

VIII,

worin $R^1$-$R^5$ die angegebene Bedeutung besitzen, mit Säure behandelt

Die Umwandlung der Verbindungen II in I gemäß a) läuft nach folgendem Schema ab:

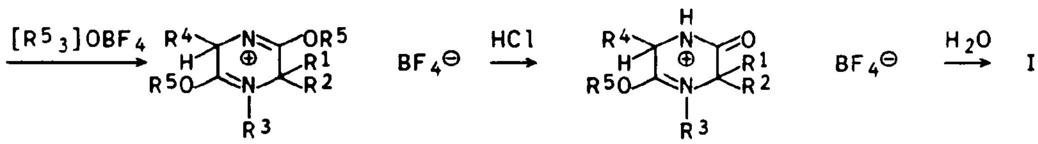


III V

Die Alkylierung der Monolactimether II wird mit Meerwein-Salzen, vorzugsweise Triethyloxonium-tetrafluoroborat oder Trimethyloxoniumtetrafluoroborat in Dichlormethan bei Raumtemperatur durchgeführt. Die Reaktionsdauer beträgt 6 bis 48 h.

Die Lösung der so entstandenen Immoniumsalze III wird bei Temperaturen von 0 bis 30°C, vorzugsweise bei Raumtemperatur mit Chlorwasserstoff gesättigt. Nach Ablauf von 5 min bis 6 h wird die Lösung von V mit Wasser versetzt, wodurch bei Raumtemperatur die Verbindungen I entstehen. Die Hydrolyse ist im allgemeinen nach 15 min bis 6 h beendet.

Die Verbindungen der Formel I, in denen $R^3$ ein Wasserstoffatom und $R^5$ eine Ethylgruppe sind, lassen sich gemäß b) besonders gut aus den Verbindungen der Formel VI

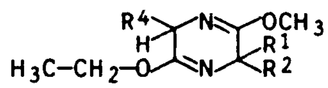

VI,

durch Umsetzen mit einem Trialkylsilyliodid, vorzugsweise mit 1 - 8 C-Atomen in den Alkylresten und insbesondere mit Trimethylsilyliodid, darstellen. Die Umsetzung wird zweckmäßigerweise bei 0 bis -20°C in einem wasserfreien Halogenkohlenwasserstoff, vorzugsweise Dichlormethan, als Lösungsmittel durchgeführt.

Die so erhaltenen Monolactimether der Formel

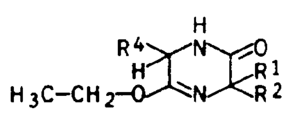

VII,

werden anschließend wie oben beschrieben zu den Verbindungen I hydrolysiert.

Die Säurespaltung der Verbindungen VIII (Verfahren c) läuft über eine Verbindung der Formel

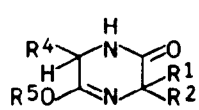

IV.

Die Spaltung erfolgt zweckmäßig mit Salzsäure bei Raumtemperatur. Will man Substanzen der Formel I herstellen, in denen $R^6$ einen Alkylrest darstellt, so muß man die Substanzen IV vor der Säurespaltung am Stickstoff alkylieren.

Da die Verbindungen der Formel I als freie Basen teilweise leicht unter Ringschluß die entsprechenden Diketopiperazine bilden, werden die bei der Hydrolyse anfallenden Salze von I vorzugsweise entweder durch Umsetzung mit den in der Peptidchemie üblichen Acylierungsmitteln wie Benzoyloxycarbonylchlorid, Fluorenyl-9-methoxycarbonylchlorid oder Di-tert.-Butylcarbonat in die N-geschützten Derivate überführt (vgl. Houben-Weyl, Methoden der organischen Chemie, Bd. 15/1, G. Thieme Verlag, Stuttgart 1974), oder mit aktivierten Aminosäurederivaten (vgl. Houben-Weyl, Methoden der organischen Chemie, Bd. 15/2, G. Thieme Verlag, Stuttgart 1974) zu Tripeptidderivaten umgesetzt.

Die Verbindungen I besitzen zwei asymmetrische C-Atome, die R- und S-konfiguriert sein können und deren Konfiguration gleich oder verschieden sein kann.

Die für die Synthese benötigten Ausgangsstoffe sind überwiegend bekannt, vgl. Pure Appl. Chem. 55, 1799 (1983), Angew. Chem. 99, 137 (1987).

Die Verbindungen VI lassen sich besonders einfach herstellen, indem man eine Verbindung der Formel

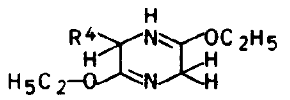

IX

mit einem Mono- oder Dialkylamin in Gegenwart eines Borsäuretrialkylesters oder einer anderen milden Lewis-Säure zu einer Verbindung der Formel

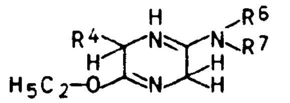

X,

worin $R^6$ ein Wasserstoffatom oder eine $C_{1-6}$-Alkylgruppe und $R^7$ eine $C_{1-6}$-Alkylgruppe darstellen, umsetzt, den Aminrest hydrolytisch entfernt und das Hydrolyseprodukt anschließend mit Trimethyloxoniumtetrafluoborat in der 6-Stellung methyliert. Die so erhaltenen gemischten Bislactimether der Formel

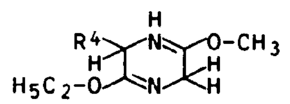

XI

können anschließend durch einfache oder doppelte C-Alkylierung ihrer Lithiumsalze in die Zwischenprodukte VI überführt werden.

Die Umsetzung von IX zu X erfolgt bevorzugt durch Reaktion von IX mit einer methanolischen Lösung von etwa 1,5 Äquivalenten Mono- oder Dialkylamin in Anwesenheit katalytischer Mengen einer Lewis-Säure bei 20 bis 60°C. Die Verbindungen X werden durch Destillation gereinigt.

Die Umsetzung von X zu XI verläuft in zwei Schritten. Die Aminogruppe wird im ersten Schritt durch Hydrolyse abgespalten. Hierzu wird X in Wasser bei pH 6 bis 9 bei 50 bis 100°C gerührt. Anschließend wird mit einem Lösungsmittel wie Halogenkohlenwasserstoff oder Essigsäureethylester extrahiert und der erhaltene Monolactimether mit Trimethyloxoniumtetraborat alkyliert. Die Alkylierung wird in einem Chlorkohlenwasserstoff bei Raumtemperatur durchgeführt und dauert in der Regel 1 bis 3 Tage. Im Anschluß an die Alkylierung wird die Lösung neutralisiert und die Verbindung XI extrahiert.

Das erfindungsgemäße Verfahren zur Herstellung der α-alkylverzweigten Dipeptide zeichnet sich dadurch aus, daß der Aufbau der unnatürlichen Aminosäure an einem ohne Schwierigkeiten zugänglichen Lactimetherderivat, d.h. einem maskierten Dipeptidderivat, erfolgt. Dieser chirale "Hilfsstoff" II wird Bestandteil des gewünschten Dipeptids. Durch die Erfindung wird es möglich, in den Verbindungen II selektiv nur eine der beiden Sollbruchstellen zu spalten. Mit Hilfe der angegebenen Reaktionsfolge gelingt überraschenderweise die selektive Hydrolyse an der Amidbindung von II in Gegenwart einer an sich labileren Lactimethergruppierung.

Verbindungen der Formel I sowie die daraus in einfacher Weise durch Umsetzung mit den üblichen Acylierungsmitteln wie Benzyloxycarbonylchlorid, Fluorenyl-9-methoxycarbonylchlorid oder Di-tert.-Butyldicarbonat ($(BOC)_2O$) herstellbaren N-geschützten Derivate dienen als Bausteine zum Aufbau biologisch aktiver Peptide. So sind z.B. eine Reihe der in EP 99 056 beschriebenen Tripeptide, die sich zur oralen Therapie des Morbus Parkinson eignen, nach dem oben beschriebenen Verfahren sehr gut zugänglich. Des weiteren eignet sich das Verfahren zur Herstellung von Bausteinen für oral wirksame Peptide bzw. Peptid-Analoga, die das Enzym Renin hemmen und daher für die Therapie des Bluthochdrucks eingesetzt werden können.

Beispiel 1

R-αMePro-L-Val-OEt

Eine Lösung von 0,45 g (2S,5R)-2,5-Dihydro-2-isopropyl-6-methoxy-5-methyl-pyrrolidino-pyrazin-3-o n in Dichlormethan wurde mit 0,76 g Triethyloxonium-tetrafluorborat unter Rühren 20 h bei Raumtemperatur umgesetzt. Anschließend wurde mit HCl-Gas gesättigt; nach 1 h wurden 5 ml wasser zugegeben. Die Hydrolyse war nach 2 h beendet. Zur Aufarbeitung wurde mit Dichlormethan (30 ml) versetzt und mit Ammoniak auf pH 9 gebrächt. Die Wasserphase wurde mit Dichlormethan (2 x 10 ml) extrahiert. Der organische Extrakt wurde getrocknet und im Vakuum eingedampft. Chromatographie des Rückstands an Kieselgel (Elution mit Dichlormethan/Ether/Ammoniak 300:100:1) ergab 0,28 g (55 %) R-αMePro-L-Val-OEt.

Beispiel 2

R-αMePhe-L-Val-OEt

1,44 g (2S,5R)-2,5-Dihydro-2-isopropyl-3,6-dimethoxy-5-benzyl-5-methyl-pyrazin in 30 ml Diethylether wurden bei Raumtemperatur mit 25 ml 0,2 N HCl in Diethylether behandelt. Nach 2 h wurde im Vakuum eingedampft. Destillation des Rückstands gab 0,94 g (69 %) rohes (2S,5R)-2,5-Dihydro-2-isopropyl-6-methoxy-5-benzyl-5-methyl-pyrazin-3-on, Kp. 170°C/0,005 Torr.

Analog Beispiel 1 wurde aus (2S,5R)-2,5-Dihydro-2-isopropyl-1-methoxy-5-benzyl-5-methyl-pyrazin-3-on durch umsetzung mit Triethyloxoniumfluoborat und anschließende Reaktion mit HCl/Dichlormethan und Hydrolyse (R)-αMePhe-L-Val-OEt erhalten.

## Patentansprüche

1. Verfahren zur Herstellung von Dipeptiden mit N-terminalen nicht-proteinogenen Aminosäuren der Formel I

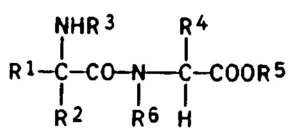

I

worin
$R^1$ eine $C_1$-$C_8$-Alkyl-, Phenyl- oder Benzylgruppe ist
$R^2$ eine $C_1$-$C_8$-Alkylgruppe, die durch -O-, -S-, -CO- oder -CO-O- unterbrochen sein kann, oder eine Phenyl- oder Benzylgruppe bedeutet,
$R^3$ ein Wasserstoffatom ist oder zusammen mit $R^2$ die Reste $-(CH_2)_3-$, $-(CH_2)_4-$ oder $-CH_2-CH=CH-CH_2-$ darstellt und
$R^4$ einen Methyl-, Isopropyl-, Isobutyl-, 2-Butyl-, t-Butyl- oder Benzylrest bedeutet,
$R^5$ eine Methyl- oder Ethylgruppe darstellt und
$R^6$ ein Wasserstoffatom oder eine $C_{1-8}$-Alkylgruppe darstellt,
wobei jedoch mindestens einer der beiden Reste $R^3$ oder $R^6$ ein Wasserstoffatom ist, welches darin besteht, daß man

a) - falls $R^6$ ein Wasserstoffatom ist - eine Verbindung der Formel II

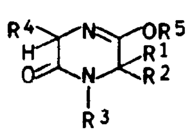

II,

worin $R^1$ bis $R^5$ die angegebene Bedeutung haben, mit Trialkyloxoniumfluoroborat zu Verbindungen der Formel III

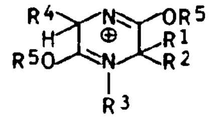 $BF_4^{\ominus}$ III,

worin $R^1$ bis $R^5$ die angegebene Bedeutung haben, umsetzt und die so erhaltenen Verbindungen mit Säure spaltet und hydrolysiert oder

b) - falls $R^3$ ein Wasserstoffatom und $R^5$ eine Ethylgruppe bedeuten - eine Verbindung der Formel IV

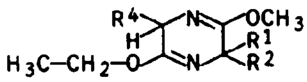 VI,

worin $R^1$, $R^2$ und $R^4$ die angegebene Bedeutung besitzen, mit einem Trialkylsilyliodid umsetzt, danach gegebenenfalls alkyliert und die so erhaltenen Verbindungen anschließend mit Säure spaltet oder

c) - falls $R^3$ ein Wasserstoffatom ist - eine Verbindung der Formel VIII

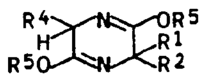 VIII,

worin $R^1$-$R^5$ die angegebene Bedeutung besitzen, mit Säure behandelt.

## Claims

1. A process for preparing dipeptides with N-terminal non-proteinogenous amino acids of the formula I

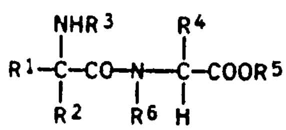 I

in which
$R^1$ is a $C_1$-$C_8$-alkyl, phenyl or benzyl group,
$R^2$ denotes a $C_1$-$C_8$-alkyl group which can be interrupted by -O-, -S-, -CO- or -CO-O-, or denotes a phenyl or benzyl group,
$R^3$ is a hydrogen atom or represents, together with $R^2$, the radicals $-(CH_2)_3-$, $-(CH_2)_4-$ or $-CH_2-CH=CH-CH_2-$, and
$R^4$ denotes a methyl, isopropyl, isobutyl, 2-butyl, t-butyl or benzyl radical,
$R^5$ represents a methyl or ethyl group, and
$R^6$ represents a hydrogen atom or a $C_1$-$C_8$-alkyl group,
but where at least one of the radicals $R^3$ or $R^6$ is hydrogen, which comprises

a) - if $R^6$ is a hydrogen atom - reacting a compound of the formula II

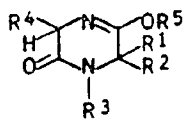 II

in which $R^1$ to $R^5$ have the stated meaning, with trialkyloxonium fluoroborate to give compounds of the formula III

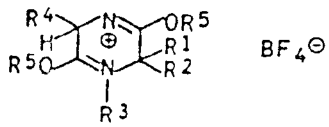

III

in which $R^1$ to $R^5$ have the stated meaning, and subjecting the compounds obtained in this way to acid cleavage and hydrolysis, or

b) - if $R^3$ denotes a hydrogen atom and $R^5$ denotes an ethyl group - reacting a compound of the formula IV

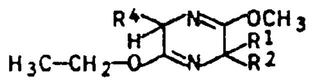

IV

in which $R^1$, $R^2$ and $R^4$ have the stated meaning, with a trialkylsilyl iodide, then alkylating where appropriate and subsequently cleaving with acid the compounds obtained in this way, or

c) - if R3 is a hydrogen atom - treating a compound of the formula VIII

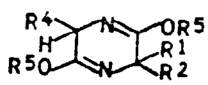

VIII

in which $R^1$-$R^5$ have the stated meaning, with acid.

## Revendications

1. Procédé de préparation de dipeptides à amino-acides non protéinogènes N-terminaux, de formule I

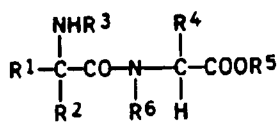

I

dans laquelle
$R^1$ est un groupe alkyle en C 1-C 8, phényle ou benzyle,
$R^2$ représente un groupe alkyle en C 1-C 8 pouvant être interrompu par -O-, -S-, -CO- ou -CO-O-, ou un groupe phényle ou benzyle,
$R^3$ représente un atome d'hydrogène ou, ensemble avec $R^2$, les restes $-(CH_2)_3-$, $-(CH_2)_4-$ ou $-CH_2-CH=CH-CH_2-$ et
$R^4$ un reste méthyle, isopropyle, isobutyle, 2-butyle, tert-butyle ou benzyle,
$R^5$ un groupe méthyle ou éthyle et
$R^6$ un atome d'hydrogène ou un groupe alkyle en C 1-C 8,
au moins un des deux restes $R^3$ ou $R^6$ étant cependant un atome d'hydrogène, caractérisé par le fait que l'on fait réagir

a) - dans le cas où $R^6$ est un atome d'hydrogène -un composé de formule II

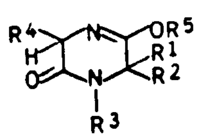

II,

dans laquelle $R^1$ à $R^5$ ont les significations données plus haut, avec du fluoroborate de trialkyloxonium pour obtenir des composés de formule III

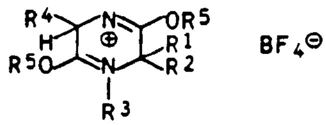

III,

dans laquelle $R^1$ à $R^5$ ont les significations données plus haut, et on dissocie les composés résultants avec un acide et les hydrolyse, ou

b) - dans le cas où $R^3$ représente un atome d'hydrogène et $R^5$ un groupe éthyle - on fait réagir un composé de formule IV

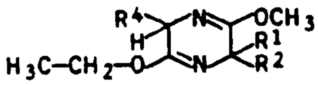

VI,

dans laquelle $R^1$, $R^2$ et $R^4$ ont les significations données plus haut, avec un iodure de trialkylsilyle, ensuite on alkyle éventuellement puis on dissocie les composés résultants avec un acide, ou

c) - dans le cas où $R^3$ est un atome d'hydrogène - on traite un composé de formule VIII

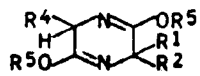

VIII,

dans laquelle $R^1$ à $R^5$ ont les significations données plus haut, avec un acide.